# EUROPEAN PATENT SPECIFICATION

(11) **EP 4 306 063 B1**
(45) Date of publication and mention of the grant of the patent: **06.05.2026**
(21) Application number: 21854795.8
(22) Date of filing: 10.03.2021
(51) Int. Cl.: A61B 17/32, A61B 1/00, A61B 17/16

(54) **SURGERY SYSTEM AND CONTROL DEVICE FOR OPERATING SURGERY SYSTEM**
CHIRURGISCHES SYSTEM UND STEUERVORRICHTUNG FÜR EIN CHIRURGISCHES SYSTEM
SYSTÈME D'OPÉRATION CHIRURGICALE ET DISPOSITIF DE COMMANDE

(43) Date of publication of application: 17.01.2024
(73) Proprietor: Olympus Corporation, Hachioji-shi Tokyo 192-8507 (JP)
(72) Inventor: WATANABE, Koichiro, Tokyo (JP); TERAYAMA, Kazuma, Tokyo (JP); YAJI, Tsuyoshi, Tokyo (JP); KOBAYASHI, Misato, Tokyo (JP)
(74) Representative: Gunzelmann, Rainer
(86) International application number: PCT/JP2021/009674
(87) International publication number: WO 2022/190296

(56) References cited:
- WO-A1-2016/171014
- JP-A- 2008 510 586
- JP-A- 2015 163 172
- JP-Y2- H0 642 641
- US-A1- 2012 190 956
- US-A1- 2018 055 568
- US-A1- 2018 271 615
- US-A1- 2018 361 055
- US-A1- 2020 016 404
- US-B2- 10 213 545

## Description

### Field

The present invention relates to a surgical system, and a control device for the surgical system.

### Background

Arthroscopic surgery is surgery in which a portal is formed in a treatment target joint, an arthroscope or a treatment tool is inserted into the treatment target joint through the portal, and a treatment is performed while observing the inside of a joint cavity by using the arthroscope in a situation where the inside of the joint cavity is filled with a perfusate.

WO 2018/078830 A (Patent Literature 1) is disclosed as an arthroscopic surgical system used for the arthroscopic surgery. In addition, Patent Literature 1 discloses an ultrasonic treatment tool for forming a hole in a bone. The ultrasonic treatment tool is configured in such a manner that a distal end of the treatment tool ultrasonically vibrates. When an operator brings the distal end of the treatment tool into contact with the bone and presses a switch to apply ultrasonic vibration to the treatment tool, the distal end of the treatment tool cuts the bone, and a hole is formed in the bone. When the distal end of the treatment tool cuts the bone, debris of the bone (bone powder) is generated.

US 2018/0055568 A1 (Patent Literature 2) discloses a system for controlling irrigation flow of a lithotripsy system wherein an irrigation flow rate is set based on received feedback by increasing the irrigation flow rate when image processing data indicates that an image is blurry and by decreasing the irrigation flow rate when image processing data indicates that the image is clear.

### Citation List

### Patent Literature

Patent Literature 1: WO 2018/078830 A
Patent Literature 2: US 2018/0055568 A1

### Summary

### Technical Problem

However, the bone powder generated at the time of cutting is temporarily dispersed in the perfusate, the perfusate becomes turbid, and the treatment target may be blocked from view of the arthroscope for observing the treatment target. In this case, the operator must stop his/her hand, which may impose a burden on a patient and the operator.

The present invention has been made in view of the above, and an object of the present invention is to provide a surgical system and a control device, for the surgical system that reduce a burden on a patient and an operator by detecting turbidity of a perfusate and performing a predetermined control.

### Solution to Problem

To solve the problem described above and to achieve the object, a surgical system and a control device as claimed in claims 1, 17 and 18 according to the present invention are provided. Further embodiments of the invention are defined by the dependent claims. The present disclosure provides a treatment tool device configured to perform a treatment on a biological tissue in a liquid; a detector configured to detect information regarding turbidity in the liquid occurring due to the treatment tool device; and a controller configured to control the surgical system based on a detection result of the detector.

A control device according to the present disclosure is a control device of a surgical system configured to supply a perfusate to a treatment target and perform a treatment on a biological tissue in the perfusate. The control device includes: a display device that is connectable to an endoscope device or a treatment tool device; and at least one control device. The control device is configured to receive information from the endoscope device or the treatment tool device via the display device, detect whether or not the perfusate is turbid based on the information, proceed to a first control when the perfusate is turbid, and proceed to a second control when the perfusate is not turbid.

An operation method for a surgical system according to the present disclosure is an operation method for a surgical system that includes an endoscope device, a perfusion device configured to supply a perfusate to a treatment target, and a treatment tool device configured to perform a treatment on a biological tissue in the perfusate in the treatment target. The operation method includes: detecting whether or not the perfusate is turbid; proceeding to a first control when the perfusate is turbid; and proceeding to a second control when the perfusate is not turbid.

### Advantageous Effects of Invention

With the surgical system and the control device according to the present invention, it is possible to reduce the burden on the patient and the operator.

### Brief Description of Drawings

FIG. 1 is a diagram illustrating a schematic configuration of a surgical system according to an embodiment.
FIG. 2 is a diagram illustrating a treatment flow using the surgical system according to the embodiment.
FIG. 3 is a diagram illustrating a cutting treatment flow using the surgical system according to the embodiment.
FIG. 4A is a diagram illustrating an example of a turbidity detection flow using the surgical system according to the embodiment.
FIG. 4B is a diagram illustrating an example of a turbidity detection flow using the surgical system according to the exemplary embodiment.
FIG. 5A is a diagram illustrating a relationship between an endoscopic visual field and a contrast value according to a first embodiment of turbidity detection.
FIG. 5B is a diagram illustrating an example of a condition for the turbidity detection according to the first embodiment of the turbidity detection.
FIG. 5C is a diagram illustrating an example of a condition for the turbidity detection according to the first embodiment of the turbidity detection.
FIG. 6A is a diagram illustrating a relationship between an endoscopic visual field and an edge according to a first modified example of the first embodiment of the turbidity detection.
FIG. 6B is a diagram illustrating an example of a condition for the turbidity detection according to the first modified example of the first embodiment of the turbidity detection.
FIG. 6C is a diagram illustrating an example of a condition for the turbidity detection according to the first modified example of the first embodiment of the turbidity detection.
FIG. 7A is a diagram illustrating a relationship between an endoscopic visual field and a luminance according to a second modified example of the first embodiment of the turbidity detection.
FIG. 7B is a diagram illustrating an example of a condition for the turbidity detection according to the second modified example of the first embodiment of the turbidity detection.
FIG. 7C is a diagram illustrating an example of a condition for the turbidity detection according to the second modified example of the first embodiment of the turbidity detection.
FIG. 8A is a diagram illustrating a relationship between an endoscopic visual field and an image difference according to a third modified example of the first embodiment of the turbidity detection.
FIG. 8B is a diagram illustrating an example of a condition for the turbidity detection according to the third modified example of the first embodiment of the turbidity detection.
FIG. 9A is a diagram illustrating an example of a turbidity detection flow using a surgical system according to a second embodiment of the turbidity detection.
FIG. 9B is a diagram illustrating an example of a turbidity detection flow using the surgical system according to the second embodiment of the turbidity detection.
FIG. 9C is a diagram illustrating an example of a condition for the turbidity detection according to the second embodiment of the turbidity detection.
FIG. 9D is a diagram illustrating an example of a condition for the turbidity detection according to the second embodiment of the turbidity detection.
FIG. 10A is a diagram illustrating an example of a condition for the turbidity detection according to a third embodiment of the turbidity detection.
FIG. 10B is a diagram illustrating an example of a condition for the turbidity detection according to the third embodiment of the turbidity detection.
FIG. 11 is a diagram illustrating an example of endoscope display according to a first embodiment of a control for coping with turbidity.
FIG. 12 is a diagram illustrating a surgical system according to a third embodiment of the control for coping with turbidity.
FIG. 13 is a diagram illustrating a surgical system according to a first modified example of the third embodiment of the control for coping with turbidity.
FIG. 14 is a diagram illustrating a surgical system according to a second modified example of the third embodiment of the control for coping with turbidity.

### Description of Embodiments

Hereinafter, embodiments for carrying out the present invention (hereinafter, referred to as the embodiments) will be described with reference to the drawings. Note that the present invention is not limited to the embodiments described below. Further, in the description of the drawings, the same reference signs denote the same parts.

### [Outline of Surgical System]

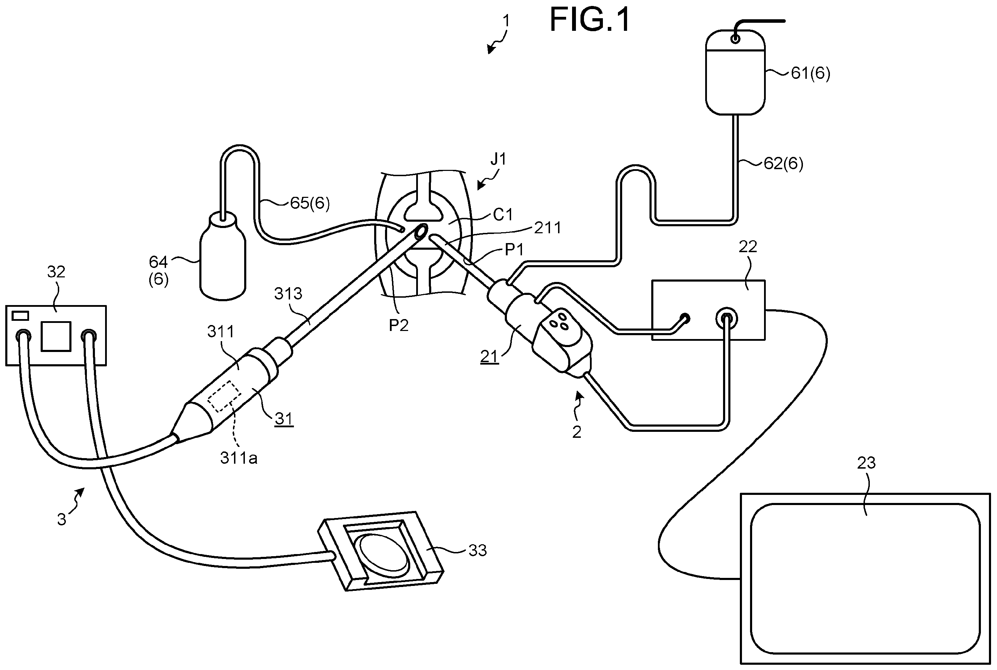

A surgical system 1 according to the present embodiment includes a treatment tool device 3 (FIG. 1).

Furthermore, the surgical system 1 includes an endoscope device 2, the treatment tool device 3, and a perfusion device 6. An operator can perform anterior cruciate ligament reconstruction surgery by using the surgical system 1.

The endoscope device 2 includes an endoscope 21, a first control device 22, and a display device 23 (FIG. 1).

A part of an insertion unit 211 of the endoscope 21 is inserted into a joint cavity C1 through a first portal P1 through which the inside of the joint cavity C1 of a knee joint J1 communicates with the outside of the skin. Then, the endoscope 21 takes in illumination light (subject image) radiated to the inside of the joint cavity C1 and reflected from the inside of the joint cavity C1 to capture the subject image.

The first control device 22 is connected to the endoscope 21 and the display device 23 in a wired or wireless manner. The first control device 22 performs various types of image processing on imaging data captured by the endoscope 21, and causes the display device 23 to display the captured image subjected to the image processing.

The treatment tool device 3 includes a treatment tool 31, a second control device 32, and a foot switch 33 (FIG. 1).

The treatment tool 31 includes a main body 311, an ultrasound probe (not illustrated), and a sheath 313 (FIG. 1). Parts of the sheath 313 and the ultrasound probe of the treatment tool 31 are inserted into the joint cavity C1 through a second portal P2 through which the inside of the joint cavity C1 of the knee joint J1 communicates with the outside of the skin.

The main body 311 is formed in a cylindrical shape. Further, an ultrasound transducer 311a that is implemented by a bolt-clamped Langevin-type transducer and generates ultrasonic vibration according to a supplied drive power is housed inside the main body 311.

The second control device 32 supplies the drive power to the ultrasound transducer 311a in response to an operation of the foot switch 33 or the like performed by the operator.

The perfusion device 6 includes a liquid source 61, a liquid feeding tube 62, a drainage bottle 64, and a drainage tube 65 (FIG. 1).

The liquid source 61 stores a perfusate. Examples of the liquid source 61 include a sterile pack of physiological saline. The liquid feeding tube 62 has one end connected to the liquid source 61 and the other end connected to the endoscope 21. Furthermore, the liquid source 61 is fixed at a position higher than the endoscope 21, such that the perfusate is fed into the joint cavity C1 via the liquid feeding tube 62. As a result, the inside of the joint cavity C1 can be filled with the perfusate.

Meanwhile, the drainage tube 65 and the drainage bottle 64 are provided to discharge the perfusate in the joint cavity C1. The drainage bottle 64 is connected to the drainage tube 65 and stores the perfusate or the like in the joint cavity C1 discharged through the drainage tube 65. Furthermore, the drainage bottle 64 is fixed at a position lower than the joint cavity C1, such that the perfusate or the like is discharged to the outside of the joint cavity C1 via the drainage tube 65.

Note that, although the embodiment illustrated in FIG. 1 has a configuration in which the first control device 22 and the second control device 32 are provided, one control device that can be connected to the endoscope 21 and the treatment tool 31 and control each of the endoscope 21 and the treatment tool 31 may be provided in another embodiment.

### [Treatment Flow]

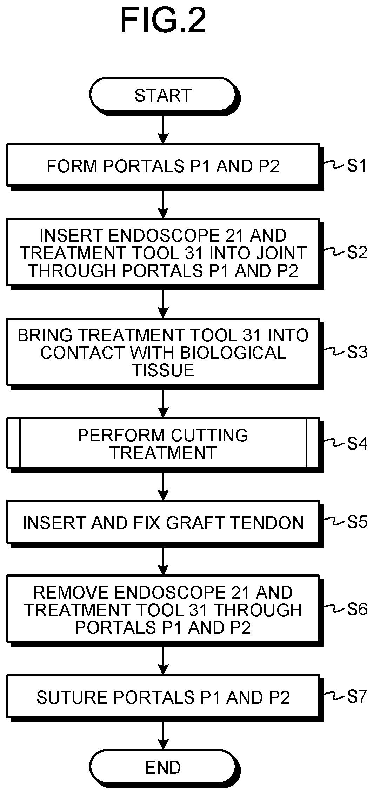

A treatment flow performed by the operator using the surgical system 1 will be described with reference to FIG. 2.

The operator forms the first portal P1 and the second portal P2 through which the inside of the joint cavity C1 of the knee joint J1 communicates with the outside of the skin (S1). Next, the operator inserts the endoscope 21 and the treatment tool 31 into the joint cavity C1 through the first portal P1 and the second portal P2, respectively (S2).

In the above description, the two portals are formed, and then the endoscope 21 and the treatment tool 31 are inserted. However, a case where the endoscope 21 is inserted after forming the first portal P1, and then the treatment tool 31 is inserted after forming the second portal P2 is also possible.

Next, the operator brings the ultrasound probe of the treatment tool 31 into contact with a treatment target while checking a state in the joint cavity C1 imaged by the endoscope 21 on the display device 23 (S3).

After the treatment tool 31 is brought into contact with the treatment target, a cutting treatment is performed (S4).

A bone hole into which a graft tendon can be inserted is formed. The graft tendon is inserted into the formed bone hole and fixed (S5).

Thereafter, the endoscope 21 and the treatment tool 31 are removed from the first portal P1 and the second portal P2, respectively (S6), the first portal P1 and the second portal P2 are sutured (S7), and the treatment flow performed by the operator using the surgical system 1 ends.

Although a case where the "operator" is one doctor has been described above, a doctor and an assistant may serve as the "operator" in a cooperative manner as necessary.

### [Cutting Treatment Flow]

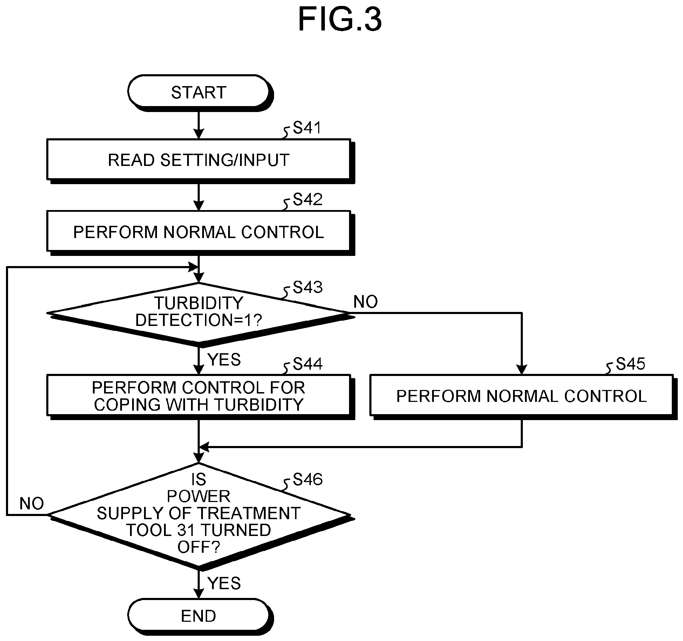

Next, a detailed flow of the cutting treatment (S4) will be described with reference to FIG. 3.

The second control device 32 reads a setting based on the ultrasound probe attached to the main body 311 (S41). A timing of reading the setting may be at the beginning of the cutting treatment, or may be immediately after a main power supply of the second control device 32 is turned on and the ultrasound probe is attached to the main body 311. Furthermore, the setting may be input in advance by the operator or the assistant.

Next, a normal control is started (S42). The normal control is a conventional control of the surgical system.

Next, the first control device 22 performs turbidity detection based on information regarding turbidity (S43).

The first control device 22 transmits a turbidity detection result to the second control device 32. In a case where the turbidity is detected ("turbidity detection = 1"), the second control device 32 proceeds to a control for coping with turbidity (S44), and in a case where the turbidity is not detected ("turbidity detection = 0"), the normal control (S45) is performed. As an exception, in a case where the operator or the assistant selects the control for coping with turbidity even when the turbidity is not detected, the processing proceeds to the control for coping with turbidity S44 similarly to the case where the turbidity is detected ("turbidity detection" = 1). The turbidity detection and the control for coping with turbidity will be described in detail later. Any combination of the turbidity detection and the control for coping with turbidity described later may be used.

After the control for coping with turbidity S44 and the normal control S45, it is checked whether or not a power supply of the treatment tool 31 is turned off (S46). In a case where the power supply is not turned off ("No"), the processing returns to the turbidity detection S43. In a case where the power supply is turned off ("Yes"), the cutting treatment ends.

### [Turbidity Detection]

Next, some specific methods for detecting the turbidity based on information regarding the turbidity will be described below. Here, the information regarding the turbidity is a value obtained from imaging data generated by the endoscope device 2, a physical property value of the perfusate, an impedance acquired from the treatment tool device 3, and the like.

### [First Embodiment of Turbidity Detection: Contrast]

A first embodiment of the turbidity detection is an embodiment in which the information regarding the turbidity is acquired from the imaging data generated by the endoscope device 2 and the turbidity is detected. The embodiment is implemented by the first control device 22 including a storage (not illustrated) that stores the imaging data generated by the endoscope device 2. The storage may be an external device. In the first embodiment of the turbidity detection, a detector is provided in the first control device 22.

Next, a detection method of the first embodiment of the turbidity detection will be described in detail. The first embodiment of the turbidity detection is a method of detecting the turbidity by using a contrast of the imaging data generated by the endoscope device 2.

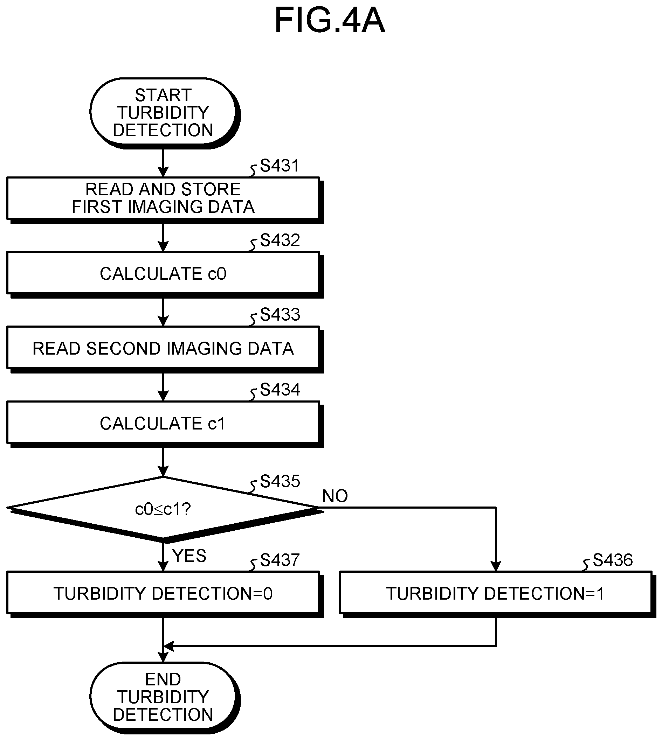

An example of the method of detecting the turbidity by using the contrast will be described with reference to FIG. 4A.

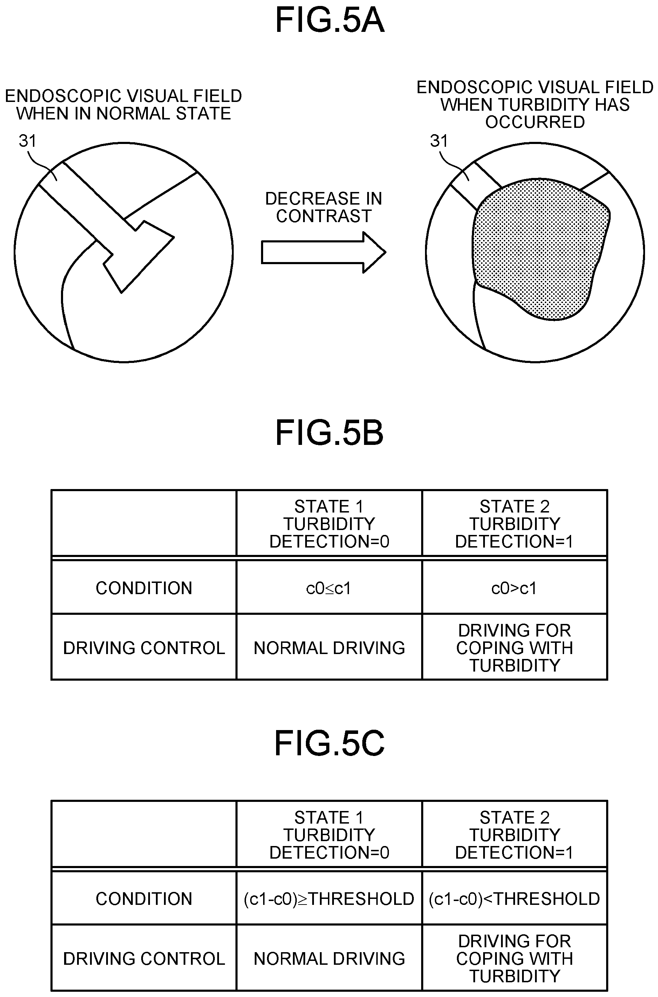

The first control device 22 reads first imaging data from the endoscope device 2 and stores the first imaging data in the storage provided in the first control device 22 (S431). Thereafter, the first control device 22 calculates a contrast c0 (first value) of the first imaging data by using a known technology (S432). Next, the detector provided in the first control device 22 reads second imaging data output from the endoscope device 2 (S433), and calculates a contrast c1 (second value) of the second imaging data by using a known technology (S434). Next, the detector provided in the first control device 22 compares the magnitudes of c0 and c1 (S435). In a case where c1 is smaller than c0, it is determined that the turbidity has been detected, and turbidity detection = 1 is set (S436: see FIG. 5B). In a case where c1 and c0 are equal to each other or c1 is larger than c0, it is determined that the turbidity has not been detected and turbidity detection = 0 is set (S437: see FIG. 5B). Once the detection result is output, the flow of the turbidity detection ends.

Note that the first imaging data is imaging data captured several seconds before or several frames before the second imaging data.

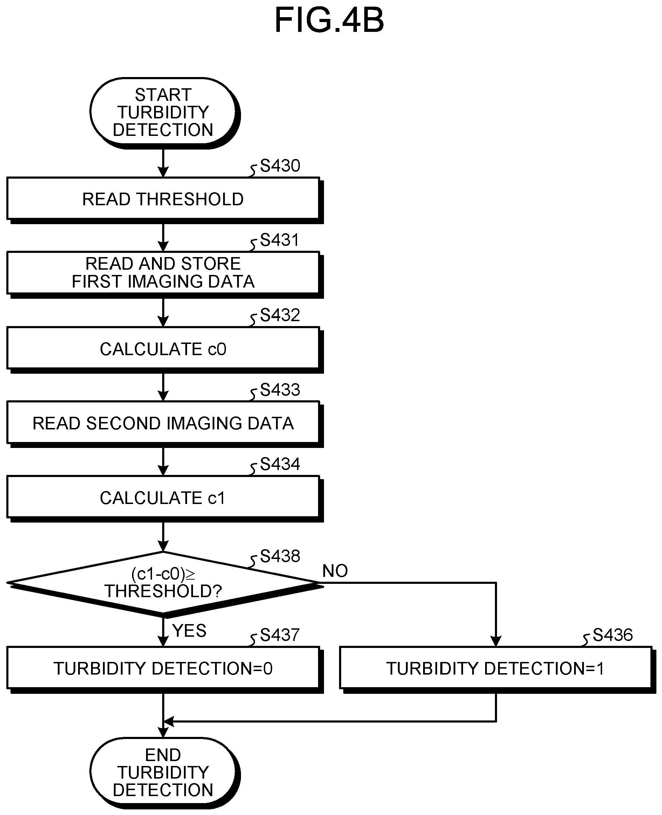

A method of detecting the turbidity by using a condition different from that of FIG. 5B will be described with reference to FIG. 4B.

The detector provided in the first control device 22 reads a preset threshold (S430). Thereafter, the detector provided in the first control device 22 performs S431 to S434 as described above. Thereafter, the detector provided in the first control device 22 calculates a change amount based on a difference between c0 and c1, and determines whether or not the turbidity has occurred based on the calculated change amount (S438: see FIG. 5C).

Here, a predetermined numerical value may be set as the threshold, or a threshold calculated by collecting images in which the turbidity has occurred and images in which the turbidity has not been occurred and performing machine learning may be used.

The cause of the turbidity is, for example, bone powder or emulsified cerebrospinal fluid. Therefore, the color of the turbidity itself is white. Accordingly, the contrast value is lower in an endoscopic visual field when the turbidity has occurred as compared with an endoscopic visual field when in a normal state (see FIG. 5A). As a result, the turbidity can be detected without depending on a color of an illumination of the endoscope 21.

### [First Modified Example of First Embodiment of Turbidity Detection: Edge]

Next, a first modified example of the first embodiment of the turbidity detection will be described. The first modified example of the first embodiment of the turbidity detection is a method in which the detector provided in the first control device 22 compares edges of the first imaging data and the second imaging data, and detects the turbidity based on the amount of change in edge.

Here, the first imaging data is imaging data captured several seconds before or several frames before the second imaging data.

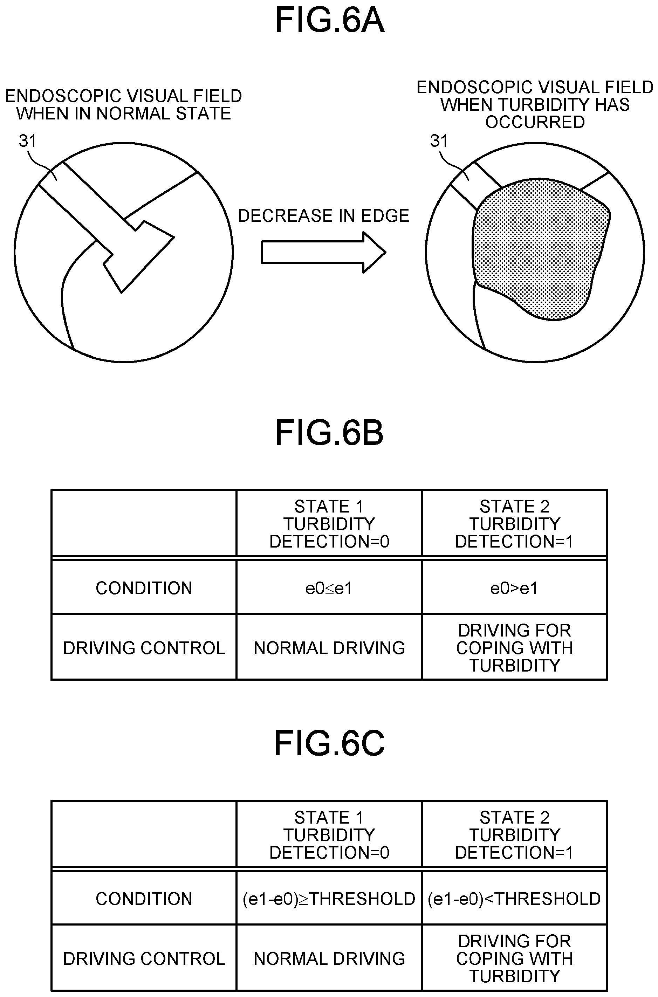

When the turbidity occurs, a biological tissue (for example, bone) and the treatment tool 31 cannot be seen. That is, an edge of the biological tissue and an edge of the treatment tool 31 cannot be imaged (see FIG. 6A). Therefore, the turbidity is detected by detecting a decrease in edge. As a result, the turbidity can be detected without depending on the color of the illumination of the endoscope.

A specific flow of the turbidity detection is similar to that in FIG. 4A. The detector provided in the first control device 22 calculates an edge e0 from the first imaging data by using a known technology instead of calculating c0 in S432. Thereafter, a condition for the turbidity detection will be described using the edge e0 and an edge e1 similarly calculated from the second imaging data by using a known technology, the edges e0 and e1 being calculated instead of determining the condition for the turbidity detection (c0 ≤ c1) used in S435.

In a case where e1 is smaller than e0, it is determined that the turbidity has occurred in the joint cavity C1, and the turbidity is detected as in S436 (see FIG. 6B). On the other hand, in a case where e1 is equal to e0 or larger than e0, it is considered that the turbidity in the joint cavity C1 has not occurred or has been decreased, and the turbidity is not detected as in S437 (see FIG. 6B).

In the above description, the values of e0 and e1 are simply compared, but a threshold may be provided as in FIG. 4B, and the turbidity may be detected by comparing a difference between e0 and e1 and the threshold (see FIG. 6C).

Here, a predetermined numerical value may be set as the threshold, or a threshold calculated by collecting images in which the turbidity has occurred and images in which the turbidity has not been occurred and performing machine learning may be used.

### [Second Modified Example of First Embodiment of Turbidity Detection: Luminance]

Next, a second modified example of the first embodiment of the turbidity detection will be described. The second modified example of the first embodiment of the turbidity detection is a method in which a luminance of the first imaging data and a luminance of the second imaging data are compared with each other and the turbidity is detected based on the amount of change in luminance.

Here, the first imaging data is imaging data captured several seconds before or several frames before the second imaging data.

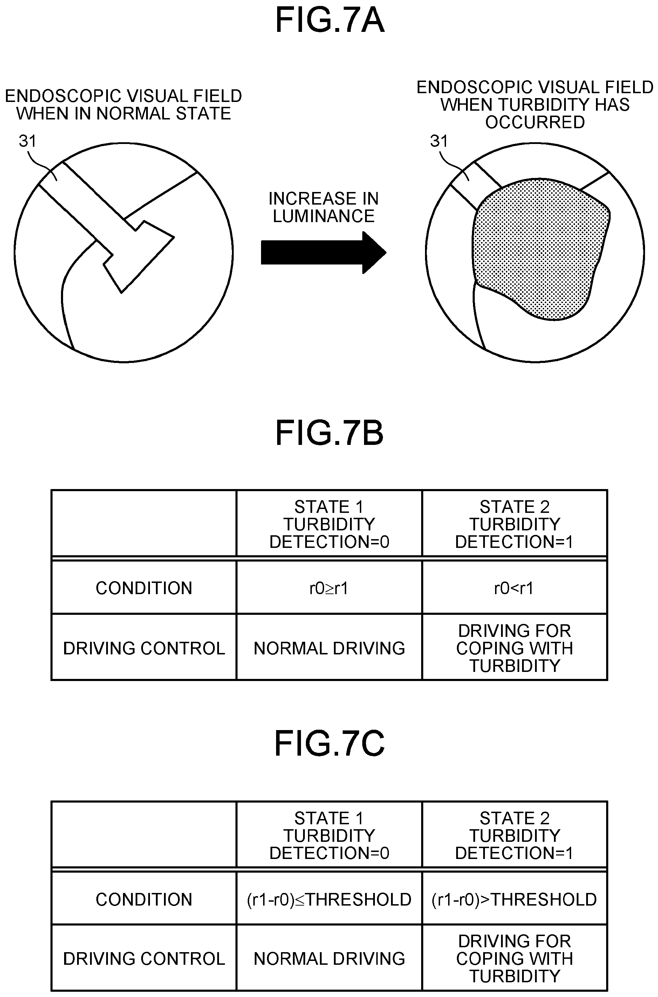

The turbidity is white as described above. Therefore, the luminance is higher when the turbidity has occurred as compared with the luminance when in the normal state (see FIG. 7A). As a result, the turbidity can be detected without depending on the color of the illumination of the endoscope.

A specific flow of the turbidity detection is similar to that in FIG. 4A. The detector provided in the first control device 22 calculates a luminance r0 from the first imaging data by using a known technology instead of calculating c0 in S432. Thereafter, a condition for the turbidity detection will be described using the luminance r0 and a luminance r1 similarly calculated from the second imaging data by using a known technology, the luminance r0 and the luminance r1 being calculated instead of determining the condition for the turbidity detection (c0 ≤ c1) used in S435.

In a case where r1 is higher than r0, it is determined that the turbidity has occurred in the joint cavity C1, and the turbidity is detected as in S436. On the other hand, in a case where r1 is equal to r0 or lower than r0, it is determined that the turbidity in the joint cavity C1 has not occurred or has been decreased, and the turbidity is not detected as in S437 (see FIG. 7B).

In the above description, the values of r0 and r1 are simply compared, but a threshold may be provided as in FIG. 4B, and the turbidity may be detected by comparing a difference (change amount) between r0 and r1 with the threshold (see FIG. 7C).

Here, a predetermined numerical value may be set as the threshold, or a threshold calculated by collecting images in which the turbidity has occurred and images in which the turbidity has not been occurred and performing machine learning may be used.

### [Third Modified Example of First Embodiment of Turbidity Detection: Image Difference]

Next, a third modified example of the first embodiment of the turbidity detection will be described. The third modified example of the first embodiment of the turbidity detection is a method in which the turbidity is detected by comparing pixels of the first imaging data and the second imaging data, calculating the amount of change in pixel, and comparing the amount of change in pixel with a threshold.

Here, the first imaging data is imaging data captured several seconds before or several frames before the second imaging data.

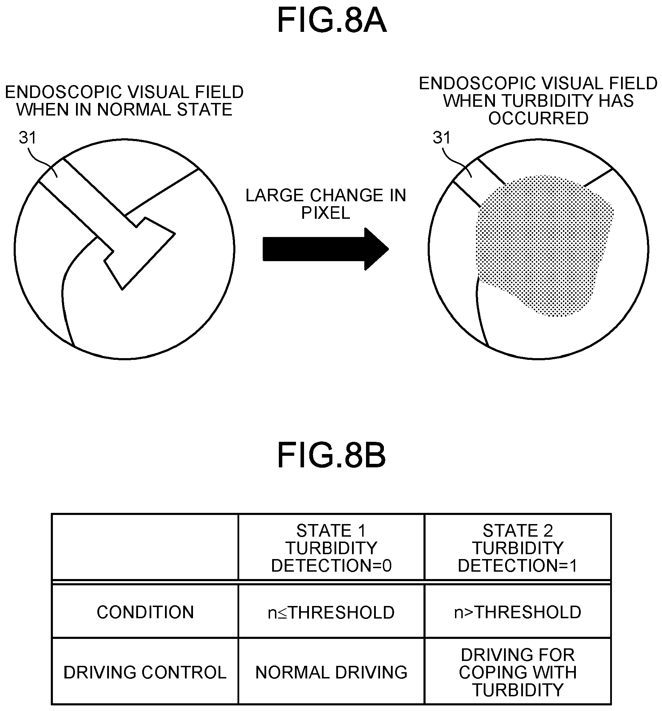

A specific flow of the turbidity detection is similar to that in FIG. 4B except for S432 and S434. The detector provided in the first control device 22 does not perform the calculation of c0 in S432, instead of calculating c1 in S434, acquires pixels of identical coordinates of the first imaging data and the second imaging data, calculates a difference between the pixels, and calculates the sum n of absolute values of the amounts of change in pixel of the respective coordinates. Thereafter, a condition for the turbidity detection will be described using the sum n of absolute values calculated instead of determining the condition for the turbidity detection (c1 - c0 ≥ threshold) used in the turbidity condition of S438 and the threshold read in S430. In a case where n is larger than the threshold, it is determined that the turbidity has occurred in the joint cavity C1, and the turbidity is detected as in S436. On the other hand, in a case where n is equal to the threshold or n is smaller than the threshold, it is determined that the turbidity in the joint cavity C1 has not occurred or has been decreased, and the turbidity is not detected as in S437 (see FIG. 8B).

When the turbidity occurs, a change appears in the pixel of the imaging data (see FIG. 8A). The turbidity can be detected by detecting the change.

### [Second Embodiment of Turbidity Detection: Turbidity Detection Based on pH of Perfusate]

A second embodiment is means for detecting the turbidity based on a physical property value of the perfusate.

The drainage bottle 64 or the insertion unit 211 of the endoscope 21 includes a pH sensor (not illustrated) that detects a pH of the perfusate. The storage may be an external device. The pH sensor provided in the drainage bottle 64 or the insertion unit 211 of the endoscope 21 is arranged at a position where the pH sensor can be in contact with the perfusate. The first control device 22 including the detector is connected to the pH sensor in a wired or wireless manner in such a manner as to be able to receive a detection value from the pH sensor provided in the drainage bottle 64 or the insertion unit 211 of the endoscope 21.

The turbidity is caused by, for example, bone, cerebrospinal fluid, or the like generated by the treatment of the treatment tool 31. Therefore, the pH of the perfusate is different between when the turbidity has not occurred and when the turbidity has occurred. It is possible to detect the turbidity due to the bone and cerebrospinal fluid by detecting the change in pH of the perfusate.

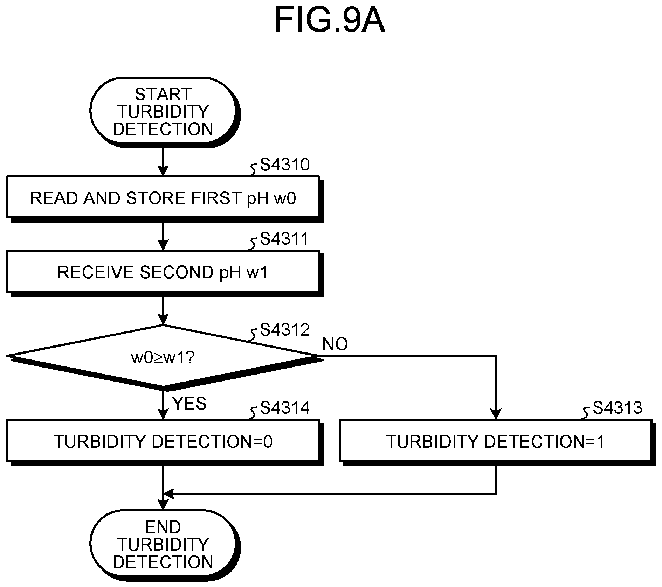

An example of a specific flow of the turbidity detection will be described with reference to FIG. 9A.

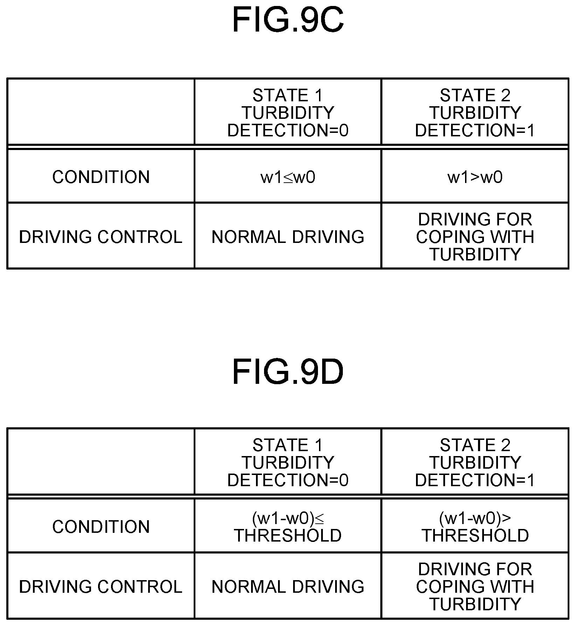

First, the detector provided in the first control device 22 reads a preset first pH w0 (S4310). Next, the detector provided in the first control device 22 receives a second pH w1 detected by the pH sensor provided in the drainage bottle 64 or the insertion unit 211 of the endoscope 21 (S4311). Thereafter, the first pH w0 stored in the detector provided in the first control device 22 is compared with the pH w1 acquired from the pH sensor provided in the drainage bottle 64 or the insertion unit 211 of the endoscope 21 (S4312). In a case where w1 is higher than w0 ("No"), it is determined that the turbidity has occurred in the joint cavity C1, and the turbidity is detected (S4313: see FIG. 9C). On the other hand, in a case where w1 is equal to w0 or lower than w0 ("Yes"), it is considered that the turbidity in the joint cavity C1 has not occurred or has been decreased, and the turbidity is not detected (S4314).

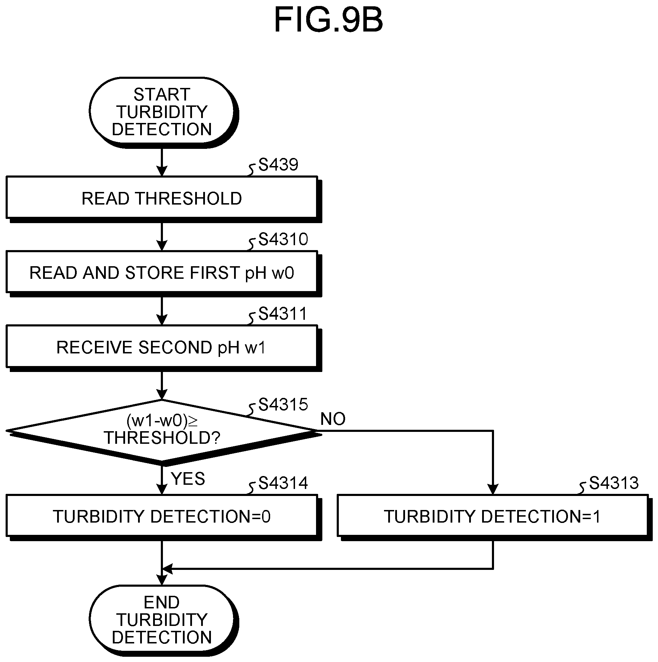

A method of detecting the turbidity by using a condition different from that of FIG. 9A will be described with reference to FIG. 9B.

The detector provided in the first control device 22 reads a preset threshold (S439). Thereafter, as described above, the detector provided in the first control device 22 performs S4310 to S4311. Thereafter, the detector provided in the first control device 22 calculates a change amount based on a difference between w0 and w1, and determines whether or not the turbidity has occurred based on the calculated change amount (S4315: see FIG. 9D).

Here, a predetermined numerical value may be set as the threshold, or a threshold calculated by collecting the pH of the perfusate when the turbidity has occurred and the pH of the perfusate when in the normal state and performing machine learning may be used.

### [Third Embodiment of Turbidity Detection: Turbidity Detection Based on Impedance (Viscosity) of Treatment Tool]

A third embodiment of the turbidity detection is a method in which the turbidity is detected based on an impedance acquired from the treatment tool 31. The method is implemented by the second control device 32 including a storage (not illustrated), and a detector is provided in the second control device 32. The storage may be an external device.

The third embodiment of the turbidity detection is a method in which the detector (not illustrated) provided in the second control device 32 calculates an impedance based on a current and a voltage supplied to perform a constant voltage control or a constant current control for the treatment tool 31 to detect the turbidity based on the calculated impedance.

The turbidity is caused by, for example, bone, cerebrospinal fluid, or the like generated by the treatment of the treatment tool 31. When a biological tissue such as bone or cerebrospinal fluid is mixed with the perfusate, a viscosity of the perfusate is changed. When the viscosity of the perfusate is changed, the impedance of the treatment tool 31 is changed. Therefore, the impedance acquired from the treatment tool 31 is different between when the turbidity has not occurred and when the turbidity has occurred. Thus, the viscosity can be predicted from a change in impedance, and the turbidity can be detected.

A specific flow of the turbidity detection is similar to that in FIG. 9A.

The detector provided in the second control device 32 reads a preset first impedance i0 instead of w0 in S4310. Thereafter, a second impedance i1 calculated at an arbitrary timing is calculated instead of w1 in S4311. A condition for the turbidity detection will be described using the read first impedance i0 and the calculated second impedance i1 instead of the turbidity detection (w0 ≥ w1) in S4312.

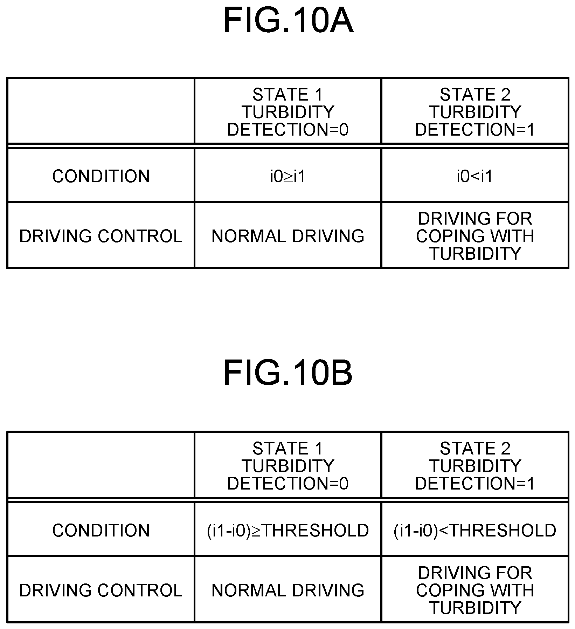

In a case where i1 is higher than i0 ("No"), it is determined that the turbidity has occurred in the joint cavity C1, and the turbidity is detected as in S4313 ("turbidity detection" = 1: see FIG. 10A). On the other hand, in a case where i1 is equal to i0 or lower than i0 ("Yes"), it is determined that the turbidity in the joint cavity C1 has not occurred or has been decreased, and the turbidity is not detected ("turbidity detection" = 0) as in S4314.

In the above description, the values of i0 and i1 are simply compared, but a threshold may be provided as in FIG. 9B, and the turbidity may be detected by comparing a difference between i0 and i1 with the threshold (see FIG. 10B).

Here, a predetermined numerical value may be set as the threshold, or a threshold calculated by collecting the impedance when the turbidity has occurred and the impedance when in the normal state and performing machine learning may be used.

### [Other Application Examples of Turbidity Detection]

The turbidity detection methods described above may be used in combination.

In a case where two types of turbidity detection are used in combination, turbidity detection = 1 may be set for a case where the turbidity has been detected in both detection methods. In this case, when any one of them detects the turbidity due to malfunction, turbidity detection = 1 is not established unless both of them detect the turbidity, such that the turbidity detection can be performed carefully.

Alternatively, turbidity detection = 1 may be set for a case where any one of the detection methods detects the turbidity. In this case, the turbidity can be detected even with a small change.

In a case where a plurality of turbidity detection methods are used in combination, the turbidity detection may be performed after weighting is performed for each detection method.

### [Control for Coping with Turbidity]

Next, a specific example of the control for coping with turbidity S44 illustrated in FIG. 3 will be described. Embodiments of the control for coping with turbidity described below can be combined with the turbidity detection described above.

Here, a controller is provided in at least one of the first control device 22 or the second control device 32, and is configured to transmit and receive a detection result of the detector in a wirelessly or wired manner. The controller performs the control for coping with turbidity based on the acquired detection result. As described above, the detection result of the detector can be transmitted to the controller, such that the controller can perform the control for coping with turbidity regardless of the arrangement of the detector.

### [First Embodiment of Control for Coping with Turbidity: Display of Edge-Superimposed Image]

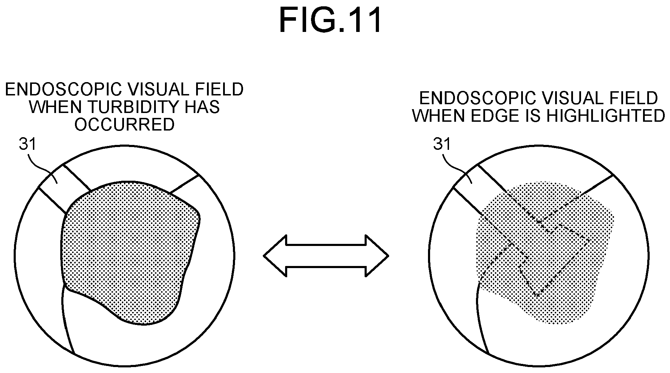

First, a first embodiment, in accordance with the present claimed invention, of the control for coping with turbidity will be described. In the first embodiment of the control for coping with turbidity, is the control for coping with turbidity in which, when the turbidity detection result = 1 is received from the detector provided in at least one of the first control device 22 or the second control device 32, an image in which the edge of the treatment tool 31 or the biological tissue is highlighted is superimposed on an endoscopic visual field image output to the display device 23 (see FIG. 11).

In the present embodiment, the controller is provided in the first control device 22.

On the other hand, in the normal control in the present embodiment, the endoscopic visual field image on which the image in which the edge is highlighted is not superimposed is output to the display device 23.

By superimposing the image in which the edge of the treatment tool 31 or the biological tissue is highlighted on the endoscopic visual field image output to the display device 23, the position of the treatment tool 31 and the position of the biological tissue can be visually recognized even in a state in which the turbidity has occurred, such that the operator can continue a surgical procedure.

Next, a flow of the first embodiment of the control for coping with turbidity will be described in detail.

When a signal for detecting the turbidity is transmitted from the detector provided in at least one of the first control device 22 or the second control device 32 to the controller provided in the first control device 22, the controller provided in the first control device 22 acquires the imaging data and extracts the edge by a known method. Thereafter, the controller provided in the first control device 22 superimposes edge-highlighted image data generated by extracting the edge on normal image data generated from the imaging data, and the display device 23 displays the superimposed image.

### [Second Embodiment of Control for Coping with Turbidity: Adjustment of Drive Power of Treatment Tool 31]

Next, a second embodiment of the control for coping with turbidity will be described. The second embodiment of the control for coping with turbidity is the control for coping with turbidity in which, when the controller receives the turbidity detection result = 1 from the detector provided in at least one of the first control device 22 or the second control device 32, drive power of the treatment tool 31 is reduced to second drive power.

In the present embodiment, the controller is provided in the second control device 32.

On the other hand, the normal control in the present embodiment is a control for driving the treatment tool 31 with first drive power that is higher than the second drive power.

A flow of the second embodiment of the control for coping with turbidity will be described in detail.

When a signal for detecting the turbidity is transmitted from the detector provided in at least one of the first control device 22 or the second control device 32 to the controller provided in the second control device 32, the controller provided in the second control device 32 adjusts the drive power to be supplied to the treatment tool 31. As a result, the treatment tool 31 ultrasonically vibrates with the second drive power lower than the first drive power.

By reducing the drive power of the treatment tool 31, a cutting speed can be reduced, and the biological tissue generated by cutting can be reduced. As a result, the speed of perfusion is not different between when the turbidity has occurred and when in the normal state, such that the turbidity in the endoscopic visual field is decreased.

Therefore, the operator can continuously perform the surgical procedure without stopping the ultrasonic vibration of the treatment tool 31.

### [First Modified Example of Second Embodiment of Control for Coping with Turbidity: Adjustment of Drive Power of Treatment Tool 31]

Next, a first modified example of the second embodiment of the control for coping with turbidity will be described. The first modified example of the second embodiment of the control for coping with turbidity is a control for coping with turbidity in which the treatment tool 31 is driven with third drive power higher than the first drive power when the turbidity is detected.

In addition, the normal control in the present modified example of the present embodiment is a control for driving the treatment tool 31 with the first drive power that is lower than the third drive power.

The first modified example of the second embodiment of the control for coping with turbidity will be described. The flow will be described in detail.

When a signal for detecting the turbidity is transmitted from the detector provided in at least one of the first control device 22 or the second control device 32 to the controller provided in the second control device 32, the controller provided in the second control device 32 adjusts the drive power to be supplied to the treatment tool 31. As a result, the treatment tool 31 ultrasonically vibrates with the third drive power higher than the first drive power.

By increasing the drive power of the cutting treatment tool, the cutting speed can be increased, and the treatment can be completed in a short time. In this method, since the amount of bone powder generated is also increased, it is more effective to set the speed of perfusion to be high.

### [Second Modified Example of Second Embodiment of Control for Coping with Turbidity: Adjustment of Drive Power of Treatment Tool 31]

Next, a second modified example of the second embodiment of the control for coping with turbidity will be described. The second modified example of the second embodiment of the control for coping with turbidity is a control for coping with turbidity in which the treatment tool 31 is repeatedly stopped and vibrated when the turbidity is detected.

In addition, the normal control in the present modification of the present embodiment is a control for intermittently driving the treatment tool 31.

A flow of the second modified example of the second embodiment of the control for coping with turbidity will be described in detail.

When a signal for detecting the turbidity is transmitted from the detector provided in at least one of the first control device 22 or the second control device 32 to the controller provided in the second control device 32, the controller provided in the second control device 32 performs a control to repeatedly cut and supply the drive power supplied to the treatment tool 31.

By repeatedly vibrating and stopping the treatment tool 31, the amount of bone powder at the time of cutting is reduced as compared with a case of continuous vibration. Therefore, it is possible to continue the surgical procedure of the operator while eliminating the turbidity in the endoscopic visual field.

### [Third Embodiment of Control for Coping with Turbidity: Perfusion Control]

Next, a third embodiment of the control for coping with turbidity will be described. The third embodiment of the control for coping with turbidity is a control in which a supply amount of the perfusion device 6 is increased (a supply speed is increased) when the turbidity is detected.

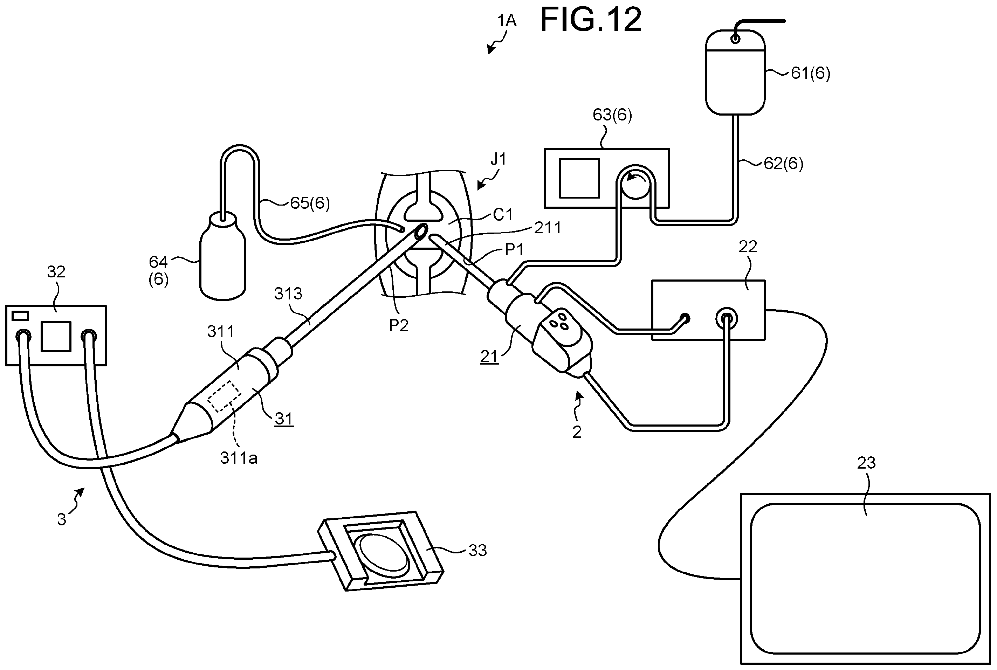

A configuration of a surgical system 1A of the present embodiment is illustrated in FIG. 12.

Only differences from the surgical system 1 will be described. A supply pump 63 is provided in the perfusion device 6.

In the present embodiment, the controller is provided in the supply pump 63 or the first control device 22.

In a case where the controller is provided in the first control device 22, the first control device 22 and the supply pump 63 are configured to transmit and receive a signal in a wireless or wired manner.

The controller provided in the supply pump 63 or the first control device 22 increases a supply amount of the perfusate flowing from the liquid source 61 toward the endoscope 21 to generate a signal as a second supply amount based on the acquired detection result to control the supply pump 63.

On the other hand, the normal control in the present embodiment is a control for supplying the perfusate in a first supply amount smaller than the second supply amount.

A flow of the third embodiment of the control for coping with turbidity will be described in detail. When a signal for detecting the turbidity is transmitted from the detector provided in at least one of the first control device 22 or the second control device 32 to the supply pump 63 or the controller provided in the first control device 22, the supply pump 63 or the first control device 22 controller performs a control to supply the perfusate in the second supply amount larger than the first supply amount.

By supplying the perfusate in the second supply amount larger than the first supply amount, the biological tissue such as the bone or cerebrospinal fluid, which is an example of the cause of the turbidity, is easily discharged from the joint cavity C1 through the drainage tube 65 by the drainage bottle 64. As a result, the turbidity in the endoscopic visual field is eliminated, and the operator can continue the surgical procedure.

### [First Modified Example of Third Embodiment of Control for Coping with Turbidity: Perfusion Control]

Next, a first modified example of the third embodiment of the control for coping with turbidity will be described. The first modified example of the third embodiment of the control for coping with turbidity is a control in which a perfusate suction amount of the perfusion device 6 is increased when the turbidity is detected.

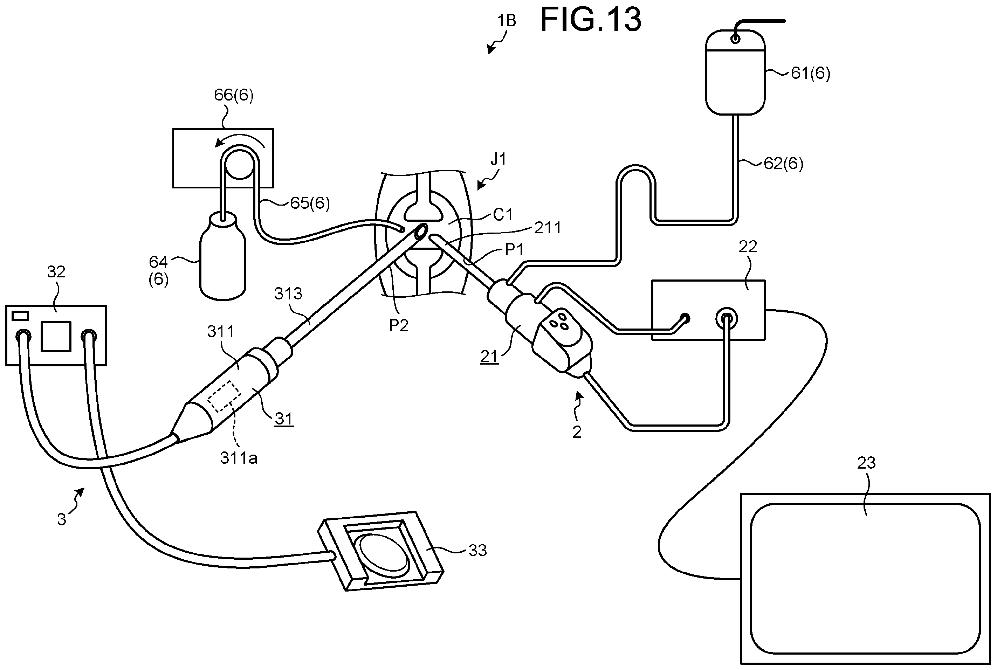

A configuration of a surgical system 1B according to the present modified example of the present embodiment is illustrated in FIG. 13. Only differences from the surgical system 1 will be described. A suction pump 66 is provided in the perfusion device 6. The suction pump 66 performs a control to increase a suction amount for discharging the perfusate in the joint cavity C1 to the drainage bottle 64 along a flow path of the drainage tube 65 to a second suction amount larger than a first suction amount.

In the present modified example of the present embodiment, the controller is provided in the suction pump 66 or the first control device 22.

In a case where the controller is provided in the first control device 22, the first control device 22 and the suction pump 66 are configured to transmit and receive a signal in a wireless or wired manner.

The controller provided in the suction pump 66 or the first control device 22 generates a signal for increasing the suction amount for discharging the perfusate in the joint cavity C1 to the drainage bottle 64 to the second suction amount based on the acquired detection result to control the suction pump 66.

On the other hand, the normal control in the present modified example of the present embodiment is a control for performing suction in the first suction amount smaller than the second suction amount.

A flow of the third embodiment of the control for coping with turbidity will be described in detail. When a signal for detecting the turbidity is transmitted from the detector provided in at least one of the first control device 22 or the second control device 32 to the suction pump 66 or the controller provided in the first control device 22, the controller provided in the suction pump 66 or the first control device 22 performs a control to increase the perfusate suction amount to the second suction amount.

By increasing the perfusate suction amount to the second suction amount, the biological tissue such as the bone or cerebrospinal fluid, which is an example of the cause of the turbidity, is more easily discharged from the joint cavity C1 to the drainage bottle 64 through the drainage tube 65. As a result, the turbidity in the endoscopic visual field is eliminated, and the operator can continue the surgical procedure.

### [Second Modified Example of Third Embodiment of Control for Coping with Turbidity: Perfusion Control]

Next, a second modified example of the third embodiment of the control for coping with turbidity will be described. The second modified example of the third embodiment of the control for coping with turbidity is a control in which the suction amount and the supply amount of the perfusate of the perfusion device 6 is increased when the turbidity is detected.

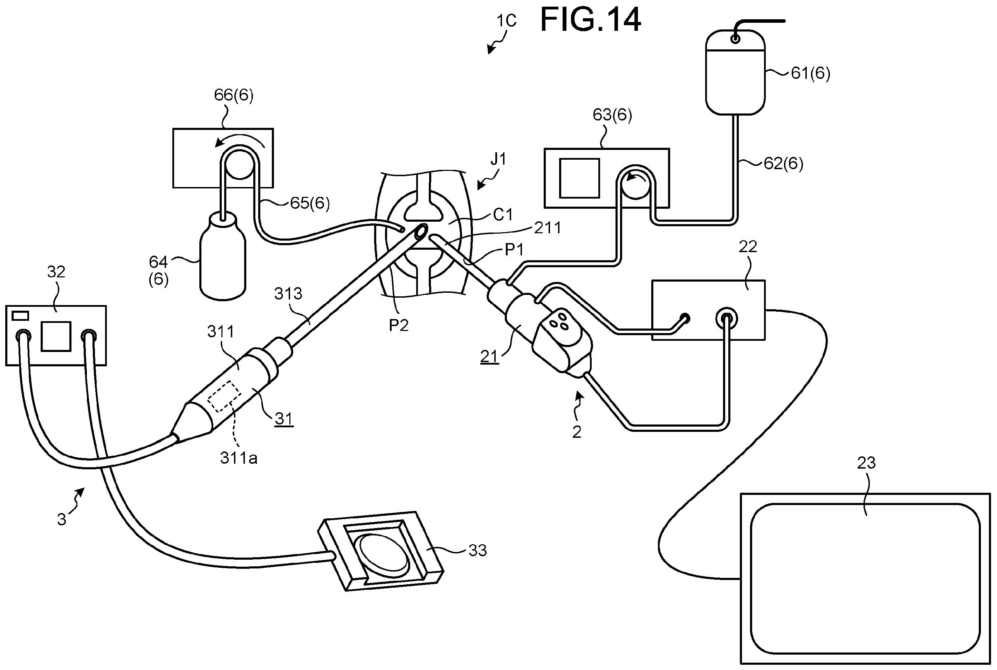

A configuration of a surgical system 1C of the present modified example of the present embodiment is illustrated in FIG. 14. Only differences from the surgical system 1 will be described. The supply pump 63 and the suction pump 66 are provided in the perfusion device 6.

In the present embodiment, the controller is provided in both the supply pump 63 and the suction pump 66, or in the first control device 22.

In a case where the controller is provided in the first control device 22, the first control device 22, the supply pump 63, and the suction pump 66 are configured to transmit and receive a signal in a wireless or wired manner.

The controller provided in both the supply pump 63 and the suction pump 66 or in the first control device 22 generates a signal for increasing the supply amount of the perfusate flowing from the liquid source 61 toward the endoscope 21 to the second supply amount based on the acquired detection result to control the supply pump 63, and generates a signal for increasing the suction amount for discharging the perfusate in the joint cavity C1 to the drainage bottle 64 to the second suction amount to control the suction pump 66.

A flow of the third embodiment of the control for coping with turbidity will be described in detail. When a signal for detecting the turbidity is transmitted from the detector provided in at least one of the first control device 22 or the second control device 32 to both the supply pump 63 and the suction pump 66 or to the controller provided in the first control device 22, both the supply pump 63 and the suction pump 66 or the controller provided in the first control device 22 performs a control to increase the supply amount of the perfusate to the second supply amount and further performs a control to increase the suction amount of the perfusate to the second suction amount.

Further, the normal control in the present modified example of the present embodiment is a control for supplying the perfusate in the first supply amount smaller than the second supply amount and a control for performing suction in the first suction amount smaller than the second suction amount.

By increasing the perfusate supply amount and the perfusate suction amount, the biological tissue such as the bone and cerebrospinal fluid, which is an example of the turbidity, is more easily discharged from the joint cavity C1 to the drainage bottle 64 through the drainage tube 65. As a result, the turbidity in the endoscopic visual field is eliminated, and the operator can continue the surgical procedure.

A control may be performed in such a manner that the perfusate supply amount and the perfusate suction amount are increased by the same amount. As a result, the amount of the perfusate in the joint cavity C1 is kept constant, and the burden on the patient is reduced.

A timing of increasing the perfusate supply amount and the perfusate suction amount may be shifted in such a manner as to increase the perfusate suction amount to the second suction amount after increasing the perfusate supply amount to the second supply amount. As a result, the amount of the perfusate in the joint cavity C1 is not temporarily reduced, and the surgical procedure is easily continued.

The timing of increasing the perfusate supply amount and the perfusate suction amount may be shifted in such a manner as to increase the perfusate supply amount to the second supply amount after increasing the perfusate suction amount to the second suction amount. As a result, the amount of the perfusate in the joint cavity C1 is not temporarily increased, and the burden on the patient is reduced.

### [Other Application Examples of Control for Coping with Turbidity]

The control for coping with turbidity described above can also be used by combining the controls for coping with turbidity. As a result, it is possible to create a situation where the operator is less likely to be interrupted.

### Industrial Applicability

As described above, the surgical system and the control device for the surgical system according to the present invention are useful for reducing the burden on the patient and the operator by detecting the turbidity of the perfusate and performing a predetermined control.

### Reference Signs List

- 1, 1A to 1C: SURGICAL SYSTEM
- 2: ENDOSCOPE DEVICE
- 3: TREATMENT TOOL DEVICE
- 6: PERFUSION DEVICE
- 21: ENDOSCOPE
- 22: FIRST CONTROL DEVICE
- 23: DISPLAY DEVICE
- 31: TREATMENT TOOL
- 32: SECOND CONTROL DEVICE
- 33: FOOT SWITCH
- 61: LIQUID SOURCE
- 62: LIQUID FEEDING TUBE
- 63: SUPPLY PUMP
- 64: DRAINAGE BOTTLE
- 65: DRAINAGE TUBE
- 66: SUCTION PUMP
- 211: INSERTION UNIT
- 311: MAIN BODY
- 311a: ULTRASOUND TRANSDUCER
- 313: SHEATH
- C1: JOINT CAVITY
- J1: KNEE JOINT
- P1: FIRST PORTAL
- P2: SECOND PORTAL

## Claims

1. A surgical system comprising:
a treatment tool device (3) configured to perform a treatment on a biological tissue in a liquid;
a detector configured to detect information regarding turbidity in the liquid occurring due to the treatment tool device;
a controller configured to control the surgical system (1) based on a detection result of the detector; and
an endoscope device (2) including:
an endoscope (21) configured to image a treatment target,
**characterized in that**
the endoscope device (2) further includes a first control device (22) configured to convert imaging data obtained from the endoscope into image data,
wherein the first control device (22) includes the detector and is configured to detect the information regarding the turbidity from the imaging data of the endoscope device (2); and
wherein the controller is configured to perform a control to highlight an edge in the imaging data.

2. The surgical system according to claim 1,
wherein the information regarding the turbidity is a value that is increased or decreased in correlation with an occurrence of the turbidity.

3. The surgical system according to claim 1,
wherein the information regarding the turbidity is information caused by the biological tissue.

4. The surgical system according to claim 2, further comprising a storage configured to store imaging data of an endoscope device (2),
wherein the detector is configured to:
acquire first imaging data from the endoscope device (2);
acquire second imaging data from the storage;
calculate an amount of change in value between pixels of identical coordinates of the first imaging data and the second imaging data; and
detect the turbidity according to the amount of change.

5. The surgical system according to claim 2, further comprising a storage configured to store imaging data of an endoscope device (2),
wherein the detector is configured to:
acquire first imaging data from the endoscope device (2);
acquire second imaging data from the storage;
calculate an amount of change in contrast between the first imaging data and the second imaging data; and
detect the turbidity according to the amount of change.

6. The surgical system according to claim 2, further comprising a storage configured to store imaging data of an endoscope device (2),
wherein the detector is configured to:
acquire first imaging data from the endoscope device (2);
acquire second imaging data from the storage;
calculate an amount of change in edge between the first imaging data and the second imaging data; and
detect the turbidity according to the amount of change.

7. The surgical system according to claim 2, further comprising a storage configured to store imaging data of an endoscope device (2),
wherein the detector is configured to:
acquire first imaging data from the endoscope device (2);
acquire second imaging data from the storage;
calculate an amount of change in luminance between the first imaging data and the second imaging data; and
detect the turbidity according to the amount of change.

8. The surgical system according to claim 1,
wherein the detector is configured to detect the information regarding the turbidity from the liquid.

9. The surgical system according to claim 8,
wherein the detector includes a sensor configured to detect a pH of the liquid, and
the detector is configured to detect the turbidity based on a value of the pH acquired from the sensor.

10. The surgical system according to claim 9,
wherein the detector is configured to detect the turbidity when the pH detected by the sensor is higher than a predetermined value.

11. The surgical system according to claim 8,
wherein the detector is configured to:
calculate an impedance of the treatment tool device (3); and
predict a viscosity of the liquid based on the calculated impedance to detect the turbidity.

12. The surgical system according to claim 1,
wherein the controller is configured to reduce drive power to be supplied to the treatment tool device (3).

13. The surgical system according to claim 1,
wherein the controller is configured to increase drive power of the treatment tool device (3).

14. The surgical system according to claim 1, further comprising a perfusion device configured to supply the liquid,
wherein the controller is configured to transmit a signal for increasing a supply speed of the liquid to the perfusion device (6).

15. The surgical system according to claim 1, further comprising a perfusion device (6) configured to suck the liquid,
wherein the controller is configured to transmit a signal for increasing a suction amount of the liquid to the perfusion device (6).

16. The surgical system according to claim 1, further comprising a perfusion device (6) configured to supply and suck the liquid,
wherein the controller is configured to transmit a signal for increasing a supply amount of the liquid to the perfusion device (6), and
the controller is configured to transmit a signal for increasing a suction amount of the liquid to the perfusion device (6).

17. A surgical system comprising:
an endoscope device (2);
a perfusion device (6) configured to supply a perfusate to a treatment target;
a treatment tool device (3) configured to perform a treatment on a biological tissue in the perfusate in the treatment target; and
a control device configured to control the endoscope device (2), the treatment tool device (3), or the perfusion device (6),
wherein the control device is configured to
detect whether or not the perfusate is turbid,
proceed to a first control when the perfusate is turbid, and
proceed to a second control when the perfusate is not turbid;
the endoscope device (2) including:
an endoscope (21) configured to image a treatment target,
**characterized in that**
the endoscope device (2) further includes a first control device (22) configured to convert imaging data obtained from the endoscope into image data,
wherein the first control device (22) includes the detector and is configured to detect the information regarding the turbidity from the imaging data of the endoscope device (2); and
wherein the control device is configured to perform a control to highlight an edge in the imaging data.

18. A control device of a surgical system configured to supply a perfusate to a treatment target and perform a treatment on a biological tissue in the perfusate, the control device comprising:
a display device that is connectable to an endoscope device (2) or a treatment tool device (3); and
at least one control device,
wherein the control device is configured to
receive information from the endoscope device or the treatment tool device via the display device,
detect whether or not the perfusate is turbid based on the information,
proceed to a first control when the perfusate is turbid, and
proceed to a second control when the perfusate is not turbid;
**characterized in that**
the control device is configured to perform a control to highlight an edge in imaging data obtained from an endoscope.

## Patentansprüche

1. Ein chirurgisches System umfassend:
eine Behandlungswerkzeugvorrichtung (3), ausgebildet, um eine Behandlung an einem biologischen Gewebe in einer Flüssigkeit durchzuführen;
einen Detektor, ausgebildet, um Informationen bezüglich einer Trübung in der Flüssigkeit, die aufgrund der Behandlungswerkzeugvorrichtung auftritt, zu detektieren;
ein Steuergerät, ausgebildet, um das chirurgische System (1) basierend auf einem Detektionsergebnis des Detektors zu steuern; und
eine Endoskopvorrichtung (2) umfassend:
ein Endoskop (21), ausgebildet, um ein Behandlungsziel abzubilden,
**dadurch gekennzeichnet, dass**
die Endoskopvorrichtung (2) ferner eine erste Steuervorrichtung (22) umfasst, die ausgebildet ist, um von dem Endoskop erhaltene Bildaufnahmedaten in Bilddaten umzuwandeln,
wobei die erste Steuervorrichtung (22) den Detektor umfasst und ausgebildet ist, um die Informationen bezüglich der Trübung aus den Bildaufnahmedaten der Endoskopvorrichtung (2) zu detektieren; und
wobei das Steuergerät ausgebildet ist, um eine Steuerung auszuführen, um eine Kante in den Bildaufnahmedaten hervorzuheben.

2. Das chirurgische System nach Anspruch 1,
wobei die Informationen bezüglich der Trübung ein Wert sind, der in Korrelation mit einem Auftreten der Trübung erhöht oder verringert wird.

3. Das chirurgische System nach Anspruch 1,
wobei die Informationen bezüglich der Trübung Informationen sind, die durch das biologische Gewebe verursacht sind.

4. Das chirurgische System nach Anspruch 2, ferner umfassend einen Speicher, der ausgebildet ist, um Bildaufnahmedaten einer Endoskopvorrichtung (2) zu speichern,
wobei der Detektor ausgebildet ist, um:
erste Bildaufnahmedaten von der Endoskopvorrichtung (2) zu erfassen;
zweite Bildaufnahmedaten von dem Speicher zu erfassen;
einen Betrag einer Änderung im Wert zwischen Pixeln gleicher Koordinaten der ersten Bildaufnahmedaten und der zweiten Bildaufnahmedaten zu berechnen; und
die Trübung gemäß dem Betrag der Änderung zu detektieren.

5. Das chirurgische System nach Anspruch 2, ferner umfassend einen Speicher, der ausgebildet ist, um Bildaufnahmedaten einer Endoskopvorrichtung (2) zu speichern,
wobei der Detektor ausgebildet ist, um:
erste Bildaufnahmedaten von der Endoskopvorrichtung (2) zu erfassen;
zweite Bildaufnahmedaten von dem Speicher zu erfassen;
einen Betrag einer Änderung im Kontrast zwischen den ersten Bildaufnahmedaten und den zweiten Bildaufnahmedaten zu berechnen; und
die Trübung gemäß dem Betrag der Änderung zu detektieren.

6. Das chirurgische System nach Anspruch 2, ferner umfassend einen Speicher, der ausgebildet ist, um Bildaufnahmedaten einer Endoskopvorrichtung (2) zu speichern,
wobei der Detektor ausgebildet ist, um:
erste Bildaufnahmedaten von der Endoskopvorrichtung (2) zu erfassen;
zweite Bildaufnahmedaten von dem Speicher zu erfassen;
einen Betrag einer Änderung einer Kante zwischen den ersten Bildaufnahmedaten und den zweiten Bildaufnahmedaten zu berechnen; und
die Trübung gemäß dem Betrag der Änderung zu detektieren.

7. Das chirurgische System nach Anspruch 2, ferner umfassend einen Speicher, der ausgebildet ist, um Bildaufnahmedaten einer Endoskopvorrichtung (2) zu speichern,
wobei der Detektor ausgebildet ist, um:
erste Bildaufnahmedaten von der Endoskopvorrichtung (2) zu erfassen;
zweite Bildaufnahmedaten von dem Speicher zu erfassen;
einen Betrag einer Änderung in Luminanz zwischen den ersten Bildaufnahmedaten und den zweiten Bildaufnahmedaten zu berechnen; und
die Trübung gemäß dem Betrag der Änderung zu detektieren.

8. Das chirurgische System nach Anspruch 1,
wobei der Detektor ausgebildet ist, um die Informationen bezüglich der Trübung aus der Flüssigkeit zu detektieren.

9. Das chirurgische System nach Anspruch 8,
wobei der Detektor einen Sensor umfasst, der ausgebildet ist, um einen pH-Wert der Flüssigkeit zu detektieren, und
der Detektor ausgebildet ist, um die Trübung basierend auf einem Wert des pH-Werts, der von dem Sensor erfasst wird, zu detektieren.

10. Das chirurgische System nach Anspruch 9,
wobei der Detektor ausgebildet ist, um die Trübung zu detektieren, wenn der von dem Sensor detektierte pH-Wert höher ist als ein vorbestimmter Wert.

11. Das chirurgische System nach Anspruch 8,
wobei der Detektor ausgebildet ist, um:
eine Impedanz der Behandlungswerkzeugvorrichtung (3) zu berechnen; und
eine Viskosität der Flüssigkeit basierend auf der berechneten Impedanz vorherzusagen, um die Trübung zu detektieren.

12. Das chirurgische System nach Anspruch 1,
wobei das Steuergerät ausgebildet ist, um eine Antriebsleistung, die der Behandlungswerkzeugvorrichtung (3) zuzuführen ist, zu verringern.

13. Das chirurgische System nach Anspruch 1,
wobei das Steuergerät ausgebildet ist, um eine Antriebsleistung der Behandlungswerkzeugvorrichtung (3) zu erhöhen.

14. Das chirurgische System nach Anspruch 1, ferner umfassend eine Perfusionsvorrichtung, ausgebildet, um die Flüssigkeit zuzuführen,
wobei das Steuergerät ausgebildet ist, um ein Signal zum Erhöhen einer Zufuhrgeschwindigkeit der Flüssigkeit an die Perfusionsvorrichtung (6) zu senden.

15. Das chirurgische System nach Anspruch 1, ferner umfassend eine Perfusionsvorrichtung (6), ausgebildet, um die Flüssigkeit abzusaugen,
wobei das Steuergerät ausgebildet ist, um ein Signal zum Erhöhen einer Absaugmenge der Flüssigkeit an die Perfusionsvorrichtung (6) zu senden.

16. Das chirurgische System nach Anspruch 1, ferner umfassend eine Perfusionsvorrichtung (6), ausgebildet, um die Flüssigkeit zuzuführen und abzusaugen,
wobei das Steuergerät ausgebildet ist, um ein Signal zum Erhöhen einer Zufuhrmenge der Flüssigkeit an die Perfusionsvorrichtung (6) zu senden, und
das Steuergerät ausgebildet ist, um ein Signal zum Erhöhen einer Absaugmenge der Flüssigkeit an die Perfusionsvorrichtung (6) zu senden.

17. Ein chirurgisches System umfassend:
eine Endoskopvorrichtung (2);
eine Perfusionsvorrichtung (6), ausgebildet, um ein Perfusat an ein Behandlungsziel zuzuführen;
eine Behandlungswerkzeugvorrichtung (3), ausgebildet, um eine Behandlung an einem biologischen Gewebe in dem Perfusat in dem Behandlungsziel durchzuführen; und
eine Steuervorrichtung, ausgebildet, um die Endoskopvorrichtung (2), die Behandlungswerkzeugvorrichtung (3) oder die Perfusionsvorrichtung (6) zu steuern,
wobei die Steuervorrichtung ausgebildet ist, um
zu detektieren, ob das Perfusat getrübt ist oder nicht,
eine erste Steuerung durchzuführen, wenn das Perfusat getrübt ist, und
eine zweite Steuerung durchzuführen, wenn das Perfusat nicht getrübt ist;
die Endoskopvorrichtung (2) umfassend:
ein Endoskop (21), ausgebildet, um ein Behandlungsziel abzubilden,
**dadurch gekennzeichnet, dass**
die Endoskopvorrichtung (2) ferner eine erste Steuervorrichtung (22) umfasst, die ausgebildet ist, um von dem Endoskop erhaltene Bildaufnahmedaten in Bilddaten umzuwandeln,
wobei die erste Steuervorrichtung (22) den Detektor umfasst und ausgebildet ist, um die Informationen bezüglich der Trübung aus den Bildaufnahmedaten der Endoskopvorrichtung (2) zu detektieren; und
wobei die Steuervorrichtung ausgebildet ist, um eine Steuerung auszuführen, um eine Kante in den Bildaufnahmedaten hervorzuheben.

18. Eine Steuervorrichtung eines chirurgischen Systems, ausgebildet, um ein Perfusat an ein Behandlungsziel zuzuführen und eine Behandlung an einem biologischen Gewebe in dem Perfusat durchzuführen, wobei die Steuervorrichtung umfasst:
eine Anzeigevorrichtung, die mit einer Endoskopvorrichtung (2) oder einer Behandlungswerkzeugvorrichtung (3) verbindbar ist; und
mindestens eine Steuervorrichtung,
wobei die Steuervorrichtung ausgebildet ist, um
Informationen von der Endoskopvorrichtung oder der Behandlungswerkzeugvorrichtung über die Anzeigevorrichtung zu empfangen,
basierend auf den Informationen zu detektieren, ob das Perfusat getrübt ist oder nicht,
eine erste Steuerung durchzuführen, wenn das Perfusat getrübt ist, und
eine zweite Steuerung durchzuführen, wenn das Perfusat nicht getrübt ist;
**dadurch gekennzeichnet, dass**
die Steuervorrichtung ausgebildet ist, um eine Steuerung auszuführen, um eine Kante in Bildaufnahmedaten hervorzuheben, die von einem Endoskop erhalten werden.

## Revendications

1. Un système chirurgical comprenant :
un dispositif d'outil de traitement (3), configuré pour effectuer un traitement sur un tissu biologique dans un fluide ;
un détecteur, configuré pour détecter des informations concernant une turbidité dans le fluide, survenant en raison du dispositif d'outil de traitement ;
une unité de commande, configurée pour commander le système chirurgical (1) sur la base d'un résultat de détection du détecteur ; et
un dispositif endoscopique (2) comprenant :
un endoscope (21), configuré pour imager une cible de traitement,
**caractérisé en ce que**
le dispositif endoscopique (2) comprend en outre un premier dispositif de commande (22), qui est configuré pour convertir des données de prise d'images obtenues par l'endoscope en données d'image,
le premier dispositif de commande (22) comprenant le détecteur et étant configuré pour détecter les informations concernant la turbidité à partir des données de prise d'images du dispositif endoscopique (2) ; et
l'unité de commande étant configurée pour exécuter une commande afin de mettre en évidence un contour dans les données de prise d'images.

2. Le système chirurgical selon la revendication 1,
les informations concernant la turbidité étant une valeur qui augmente ou diminue en corrélation avec une survenue de la turbidité.

3. Le système chirurgical selon la revendication 1,
les informations concernant la turbidité étant des informations qui sont causées par le tissu biologique.

4. Le système chirurgical selon la revendication 2, comprenant en outre une mémoire, configurée pour stocker des données de prise d'images d'un dispositif endoscopique (2),
le détecteur étant configuré pour :
acquérir des premières données de prise d'images du dispositif endoscopique (2) ;
acquérir des deuxièmes données de prise d'images de la mémoire ;
calculer une valeur absolue d'une variation de la valeur entre des pixels de mêmes coordonnées des premières données de prise d'images et des deuxièmes données de prise d'images ; et
détecter la turbidité conformément à la valeur absolue de la variation.

5. Le système chirurgical selon la revendication 2, comprenant en outre une mémoire, configurée pour stocker des données de prise d'images d'un dispositif endoscopique (2),
le détecteur étant configuré pour :
acquérir des premières données de prise d'images du dispositif endoscopique (2) ;
acquérir des deuxièmes données de prise d'images de la mémoire ;
calculer une valeur absolue d'une variation de contraste entre les premières données de prise d'images et les deuxièmes données de prise d'images ; et
détecter la turbidité conformément à la valeur absolue de la variation.

6. Le système chirurgical selon la revendication 2, comprenant en outre une mémoire, configurée pour stocker des données de prise d'images d'un dispositif endoscopique (2),
le détecteur étant configuré pour :
acquérir des premières données de prise d'images du dispositif endoscopique (2) ;
acquérir des deuxièmes données de prise d'images de la mémoire ;
calculer une valeur absolue d'une variation d'un contour entre les premières données de prise d'images et les deuxièmes données de prise d'images ; et
détecter la turbidité conformément à la valeur absolue de la variation.

7. Le système chirurgical selon la revendication 2, comprenant en outre une mémoire, configurée pour stocker des données de prise d'images d'un dispositif endoscopique (2),
le détecteur étant configuré pour :
acquérir des premières données de prise d'images du dispositif endoscopique (2) ;
acquérir des deuxièmes données de prise d'images de la mémoire ;
calculer une valeur absolue d'une variation de luminance entre les premières données de prise d'images et les deuxièmes données de prise d'images ; et
détecter la turbidité conformément à la valeur absolue de la variation.

8. Le système chirurgical selon la revendication 1,
le détecteur étant configuré pour détecter les informations concernant la turbidité à partir du fluide.

9. Le système chirurgical selon la revendication 8,
le détecteur comprenant un capteur, configuré pour détecter une valeur de pH du fluide, et
le détecteur étant configuré pour détecter la turbidité sur la base d'une valeur de la valeur de pH, acquise par le capteur.

10. Le système chirurgical selon la revendication 9,
le détecteur étant configuré pour détecter la turbidité lorsque la valeur de pH détectée par le capteur est supérieure à une valeur prédéterminée.

11. Le système chirurgical selon la revendication 8,
le détecteur étant configuré pour :
calculer une impédance du dispositif d'outil de traitement (3) ; et
prédire une viscosité du fluide sur la base de l'impédance calculée, afin de détecter la turbidité.

12. Le système chirurgical selon la revendication 1,
l'unité de commande étant configurée pour diminuer une puissance d'entraînement à fournir au dispositif d'outil de traitement (3).

13. Le système chirurgical selon la revendication 1,
l'unité de commande étant configurée pour augmenter une puissance d'entraînement du dispositif d'outil de traitement (3).

14. Le système chirurgical selon la revendication 1, comprenant en outre un dispositif de perfusion, configuré pour fournir le fluide,
l'unité de commande étant configurée pour envoyer au dispositif de perfusion (6) un signal destiné à augmenter une vitesse d'alimentation du fluide.

15. Le système chirurgical selon la revendication 1, comprenant en outre un dispositif de perfusion (6), configuré pour aspirer le fluide,
l'unité de commande étant configurée pour envoyer au dispositif de perfusion (6) un signal destiné à augmenter une quantité d'aspiration du fluide.

16. Le système chirurgical selon la revendication 1, comprenant en outre un dispositif de perfusion (6), configuré pour fournir et aspirer le fluide,
l'unité de commande étant configurée pour envoyer au dispositif de perfusion (6) un signal destiné à augmenter une quantité d'alimentation du fluide, et
l'unité de commande étant configurée pour envoyer au dispositif de perfusion (6) un signal destiné à augmenter une quantité d'aspiration du fluide.

17. Un système chirurgical comprenant :
un dispositif endoscopique (2) ;
un dispositif de perfusion (6), configuré pour fournir un perfusat à une cible de traitement ;
un dispositif d'outil de traitement (3), configuré pour effectuer un traitement sur un tissu biologique dans le perfusat dans la cible de traitement ; et
un dispositif de commande, configuré pour commander le dispositif endoscopique (2), le dispositif d'outil de traitement (3) ou le dispositif de perfusion (6),
le dispositif de commande étant configuré pour
détecter si le perfusat est trouble ou non,
exécuter une première commande lorsque le perfusat est trouble, et
exécuter une deuxième commande lorsque le perfusat n'est pas trouble ;
le dispositif endoscopique (2) comprenant :
un endoscope (21), configuré pour imager une cible de traitement,
**caractérisé en ce que**
le dispositif endoscopique (2) comprend en outre un premier dispositif de commande (22), qui est configuré pour convertir des données de prise d'images obtenues par l'endoscope en données d'image,
le premier dispositif de commande (22) comprenant le détecteur et étant configuré pour détecter les informations concernant la turbidité à partir des données de prise d'images du dispositif endoscopique (2) ; et
le dispositif de commande étant configuré pour exécuter une commande afin de mettre en évidence un contour dans les données de prise d'images.

18. Un dispositif de commande d'un système chirurgical, configuré pour fournir un perfusat à une cible de traitement et effectuer un traitement sur un tissu biologique dans le perfusat, le dispositif de commande comprenant :
un dispositif d'affichage, connectable à un dispositif endoscopique (2) ou à un dispositif d'outil de traitement (3) ; et
au moins un dispositif de commande,
le dispositif de commande étant configuré pour
recevoir, via le dispositif d'affichage, des informations provenant du dispositif endoscopique ou du dispositif d'outil de traitement,
détecter, sur la base des informations, si le perfusat est trouble ou non,
exécuter une première commande lorsque le perfusat est trouble, et
exécuter une deuxième commande lorsque le perfusat n'est pas trouble ;
**caractérisé en ce que**
le dispositif de commande est configuré pour exécuter une commande afin de mettre en évidence un contour dans des données de prise d'images obtenues par un endoscope.
